Europäisches Patentamt

European Patent Office

Office européen des brevets

(11) Publication number: **0 106 615**
**B1**

# EUROPEAN PATENT SPECIFICATION

(45) Date of publication of patent specification: **21.01.87**

(51) Int. Cl.⁴: **G 01 N 33/577**

(21) Application number: **83305988.4**

(22) Date of filing: **03.10.83**

(54) Assay for the free portion of substances in biological fluids.

(30) Priority: **07.10.82 GB 8228728**

(43) Date of publication of application:
**25.04.84 Bulletin 84/17**

(45) Publication of the grant of the patent:
**21.01.87 Bulletin 87/04**

(84) Designated Contracting States:
**BE CH DE FR GB IT LI NL SE**

(56) References cited:
**EP-A-0 015 687**
**GB-A-2 030 290**
**GB-A-2 085 160**

**CHEMICAL ABSTRACTS, vol. 89, no. 25, 18th December 1978, page 430, no. 213358x, Columbus, Ohio, US G. BUTTIN et al.: "Production of hybrid lines secreting monoclonal anti-idiotypic antibodies by cell fusion on membrane filters"**

(73) Proprietor: **AMERSHAM INTERNATIONAL plc**
**White Lion Road**
**Amersham Buckinghamshire, HP7 9LL (GB)**

(72) Inventor: **Brockas, Anthony John**
**Highmead 69, Carpenters Wood Drive**
**Chorleywood Hertfordshire, WD3 SRP (GB)**

(74) Representative: **Pennant, Pyers et al**
**Stevens, Hewlett & Perkins 5 Quality Court**
**Chancery Lane**
**London, WC2A 1HZ (GB)**

(56) References cited:

**CHEMICAL ABSTRACTS, vol. 81, no. 25, 23rd December 1974, page 390, no. 167496a, Columbus, Ohio, US J.R. NORTH et al.: "Analysis of affinity of monoclonal antibody responses by inhibition of plaque-forming cells"**

Courier Press, Leamington Spa, England.

## Description

This invention relates to an assay for the free portion of organic substances or analytes that are present in biological fluids in both a form bound to protein (or other binding species present in the fluid) and in a non-bound or free form, the free and bound forms being in equilibrium with one another.

For most physiologically active substances that can be found jointly in both a free form and a protein-bound form in biological fluids such as blood, it is currently thought that it is the concentration of the free form that may control the physiological responses associated with those substances and may therefore be more significant clinically than the concentration of total substance which includes both free (or unbound) and protein-bound substance.

European Patent Specification No. 26103 describes an assay method of this kind. The method involves causing the analyte and a labelled derivative thereof to compete for reaction with a specific binder for the analyte. The amount of the labelled derivative of the analyte which has become bound to·the specific binder is then measured, and the measurement used to determine the concentration of the free analyte in the biological fluid.

European Patent Specification No. 89806 describes another assay method of a similar kind. The method involves causing the analyte and a derivative thereof to compete for reaction with a labelled specific binder for the analyte. The amount of the derivative of the analyte which has become bound to the labelled specific binder is then measured and the measurement used to determine the concentration of the free analyte in the biological fluid.

In both the methods of European Patent Specification Nos. 26103 and 89806 the amount of specific binder used is insufficient to substantially affect the equilibrium between the free and the bound forms of the analyte. Also, the analyte derivative is chosen to be substantially non-reactive with the natural binders in the biological fluid.

A problem in the successful use of both methods lies in designing a suitable analyte derivative. A suitable analyte derivative should:—

a) be chemically modified so as to be substantially non-reactive with the natural binders in the biological fluid, although the analyte itself is reactive with such binders;

b) in the case of European Patent Specification No. 26103, be labelled with a radioactive atom or other marker, which usually involves further chemical modification;

c) in the case of European Patent Specification No. 89806, generally be linked to an insoluble matrix; and

d) nevertheless, bind strongly to the specific binder.

It is a matter of difficulty and considerable trial and error to modify an analyte molecule, especially if it is a small one, to provide a suitable analyte derivative. It is an object of the present invention to overcome these difficulties, and this object is achieved by the use, instead of an analyte derivative, of an antibody to the specific binder. When, as is often the case, the specific binder is itself an antibody to the analyte, this antibody to the specific binder may be an anti-idiotype antibody.

Anti-idiotype antibodies are known materials, whose preparation is described in various references. The nature of an anti-idiotype antibody will be explained in the case where the specific binder for the analyte is an antibody to the analyte (antigen). An antibody is a Y-shaped molecule, having antigen binding sites at the ends of the two branches of the Y. The antigen binding site is formed by the folding of the light and heavy chains constituting the arms of the Y to form a region complementary to the area of the antigen to which the antibody binds. In order to generate the wide repertoire of antibody specificities which the immune system requires there is a system of variable amino acid sequence regions (variable regions) built into the light and heavy chains of the antibody molecule. The amino acid sequence in the variable region of an antibody thus determines the specificity of that antibody, and more particularly that sequence is unique to that antibody. This unique amino acid sequence (called an idiotype) is moreover not part of the self-concept of the animal. It can therefore be recognised as foreign and can elicit an antibody response to itself. Such anti-idiotype antibodies are thought to play a part in the natural control of antibody synthesis by the immune system.

Thus, an antibody possesses a unique amino acid sequence (idiotype) in its antigen (analyte) binding site, and this can form the basis for an anti-idiotype antibody response.

Potocynjak P et al (Science 1982, 215, 1637 to 1639) describe a radio assay using an anti-idiotype antibody. The analyte (antigen) was a 44,000-dalton membrane protein (Pb 44). Monoclonal antibodies (3D11) were raised against this antigen. Monoclonal anti-idiotype antibodies (2D 12) were raised against the idiotype of the antibody 3D11 and were labelled with iodine-125. The antibody 3D11 was immobilized on the walls of assay tubes. Then, in a two-stage incubation, the antigen Pb 44 and the anti-idiotype antibody 2D12 were caused to compete for binding sites on the antibody 3D11.

The aforesaid assay was designed to measure total analyte. By contrast, the method of the present invention is designed to measure free analyte in a system in which free and bound analyte are in equilibrium. As noted below, the use of anti-idiotype antibodies gives rise to special and particular advantages in the measurement of the free portion of an analyte.

The invention

The present invention provides a competitive method of determining the concentration of the free portion of an analyte which is a member of a specific binding pair consisting of the analyte and a specific binder therefor, said free portion of the analyte being present in a biological fluid which also contains a portion of the analyte bound to one or more natural binders for the analyte, the bound and free portions of the analyte being in equilibrium with one another, by:—

a) forming a mixture of a sample of the biological fluid with an amount of the specific binder for the analyte and with a monoclonal antibody to the specific binder which competes with the analyte for binding sites on the specific binder, the amount of said specific binder being insufficient to substantially affect said equilibrium, at least one of said specific binder and said antibody being labelled:—

b) maintaining said mixture for a time to permit the free portion of the analyte and the antibody to compete for binding sites on the specific binder and become bound thereto in proportions which depend on the concentration of the free portion of the analyte present in the sample;

c) measuring the amount of the specific binder bound to its antibody, and/or the amount of the specific binder not bound to the antibody; and

d) using the measurement to determine the concentration of free analyte in the biological fluid.

This invention also includes an assay kit for use in the said method, comprising a supply of a specific binder for the analyte and a supply of an antibody to the specific binder, at least one of the specific binder and the antibody being labelled.

The principle of the invention can readily be understood by reference to the accompanying drawings, in which Figure 1 is a reaction scheme of a prior assay as described in European Patent Specification No. 26103 or 89806; and Figures 2 and 3 are reaction schemes of assays according to the present invention.

Referring to the drawings, the analyte 12 is shown in the shape of a letter t, having a box-shaped top and two U-shaped arms.

Reference 10 designates serum binding protein present in the biological fluid containing the analyte, and these are shown as having U shaped binding sites for the analyte.

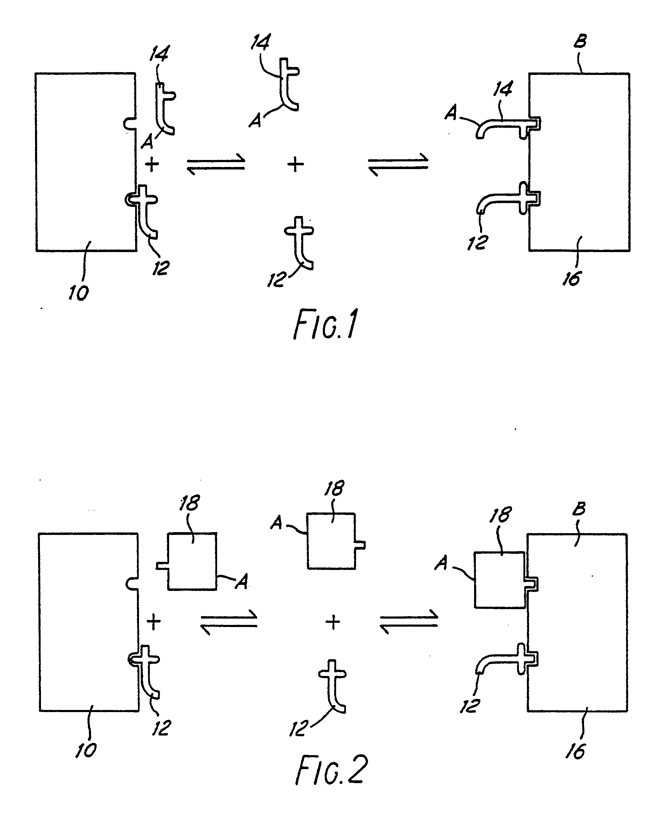

In Figure 1, the analyte derivative is designated 14 and shown as a letter t, missing one arm so that it will not bind to the serum binding protein 10.

The specific binder is designated 16, and is shown as having box-shaped binding sites for the analyte 12 and the analyte derivative 14.

In Figure 2, an antibody is designated 18, and is shown as having a box-shaped projection which is bound by the specific binder 16 but not by the serum binding proteins 10.

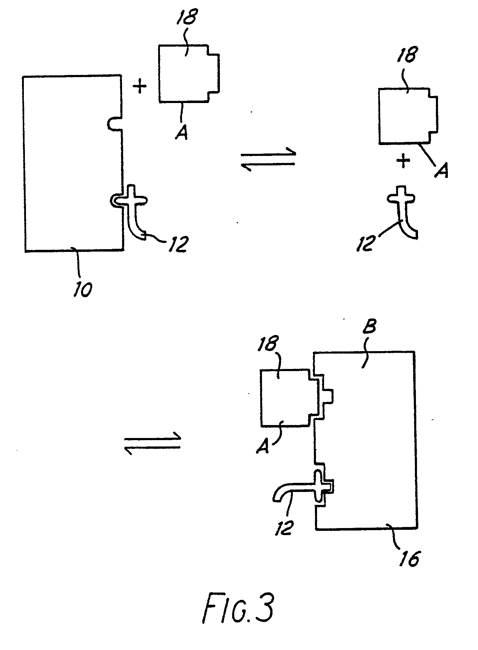

In Figure 3, the antibody 18 is shown as having a box-shaped projection broader and flatter than the box-shaped top of the t analyte 12. The specific binder 16 is shown as having two kinds of binding sites, one for the antibody 18 and the other for the analyte 12. These are so positioned in relation to each other that the binding of an antibody at one location prevents the binding of an analyte molecule at that location, and vice versa.

Designations A and B appear in all figures. In each figure, these designations have the following meanings. Either A or B signifies a label, for example a radioactive atom or fluorescent group. The other of A and B signifies a means, e.g. an insoluble matrix, by which the reagent to which it is attached may readily be removed from the reaction medium. Thus, referring to Figure 2, A attached to the antibody 18 may designate a radioactive or other label; and B attached to the specific binder 16 may designate an insoluble matrix. Alternatively, A may designate an insoluble matrix, in which case B designates radioactive or other label.

Referring to Figure 1, the inventions of the prior art were based on the idea that the analyte derivative 14 should bind to the specific binder 16 in exactly the same way as the analyte 12—that is, the region of the analyte which binds to the specific binder should not be modified when the analyte molecule as a whole is modified to prevent binding to natural protein binders 10.

However, it is not necessary that the two molecules should bind in exactly the same way to the specific binder. All that is necessary is that, if the analyte molecule is bound to the specific binder, then the other molecule should not bind and vice versa, so that there is competition for the specific binder.

Referring to Figure 3, this leads to another approach, namely the use of an antibody 18 which binds to the specific binder 16 in a region sufficiently close to the binding site of the analyte 12 to interfere with binding of the analyte molecule, and vice versa.

The use of an antibody to the specific binder according to this invention brings advantages:—

a) Antibodies as a class do not bind to serum proteins. There is therefore no significant risk that the antibody used will bind to the serum binding proteins of the biological fluid containing the analyte. This is an important advantage in assays for free analyte, but is not significant where total analyte is being assayed, as was the case in the paper of Potocynjak et al referred to above.

b) The antibody is a sufficiently large molecule to permit labelling or attachment to an insoluble matrix, without danger of interference with binding of the specific binder.

The analyte

As regards the nature of the analyte, the method of the invention is of general applicability. Examples of classes of analytes to which the method may be applied are hormones, biochemical messengers, steroids, drugs, drug metabolites, polypeptides, proteins, vitamins, tumour antigens, toxins, alkaloids and mono-, di- and poly-saccharides. The invention is particularly advantageous in relation to small analytes, because it is particularly difficult to modify small analyte molecules without interfering with their binding to specific binders.

The analyte is a member of a specific binding pair. When the analyte is a hapten or is itself immunogenic, the specific binder therefor may conveniently be an antibody to the analyte. But the invention is not confined to antibodies as specific binders, and is applicable to specific binding pairs generally, for example to the vitamin B12-intrinsic factor system.

The free portion of the analyte is in equilibrium with the portion of the analyte bound to the natural binders in the biological fluid. Thus, if a small amount of free analyte is removed from the system (e.g. by becoming bound to its specific binder), then a corresponding small amount of analyte is freed by the natural binders in the biological fluid, so as to restore the equilibrium. Moreover, this process takes place in a time generally less than, and in any event not substantially greater than, the time taken for the assay.

The specific binder

The specific binder should have a high affinity for the analyte, and that affinity should desirably be as high as, or more preferably higher than, that of any natural protein binder present in the biological fluid being assayed. As an approximate rule of thumb, the dissociation constant of the analyte/specific binder complex should preferably be within a factor of 10 greater or less than the concentration of the free analyte (in moles per litre) in the assay sample. If the specific binder has too low an affinity for the analyte, then the sensitivity of the assay is reduced and it may be necessary to use a larger quantity of specific binder. Also, the specific binder must be able to discriminate between the analyte and related substances likely to be present in the biological fluid. When, as is frequently the case, the specific binder is an antibody to the analyte, the antibody is preferably monoclonal. If a mixture of antibodies (all having affinity for the analyte) were used, such as might be obtained in a standard anti-serium, then a mixture of anti-idiotype antibodies would be needed. Monoclonal antibodies may be prepared by hyridoma techniques which are well described in the literature.

According to one aspect of the invention, the specific binder may be labelled with any label which is used in immunoassays or immunometric assays. The most important groups of labels are radioactive atoms, enzymes or components of enzyme systems, and chemiluminescent and fluorescent groups. An enzyme assay involves the use of two or more components, e.g. an enzyme and its substrate, to generate a signal, and the specific binder may have been labelled with one or more of these components. Most usually, the specific binder will have been labelled with at least one radioactive atom, preferably one which emits $\gamma$-radiation. Techniques for labelling antibodies and other specific binders are well known in the art and will not be described here.

In another embodiment of the invention, it is the antibody which is labelled. In this case, the specific binder may conveniently be provided with means for its easy removal from the reaction medium, or may be used in an insoluble form. For instance, the specific binder may be adsorbed on insoluble particles or on the wall of the assay tube, prior to performing the assay.

In a conventional competition assay the specific binder for the analyte is present in an amount insufficient to react with all of the analyte and its derivative. At first sight, therefore, it might appear that, in the present invention, the specific binder should be present in an amount insufficient to react with all of the free analyte and the antibody.

This is not so. Because some of the free analyte is removed by the specific binder, more analyte is removed from the natural binders to take its place. It may be advantageous to employ an amount of specific binder greater than that required to react with all the free analyte initially present and the antibody. Clearly an upper limit is set by the amount of specific binder which would react with all of the analyte present, whether free or bound to natural binders, plus the antibody, but this is not a very helpful indication for practical use.

It is to be recognised that at equilibrium the proportion of the specific binder bound to the antibody will be determined by the amounts of natural binders and specific binder present, by their affinity constants for the analyte and for the antibody, and by the amounts of analyte and antibody. While it might be theoretically possible to calculate the amount of analyte in the free form from a knowledge of this proportion and other relevant data, this is not a practicable procedure, and recourse must be made to a standard assay procedure, namely the use of a "dose-response curve" or "standard curve". In this procedure, a number of standard sera of known free analyte content (determined, for example, by equilibrium dialysis), spanning the required working range of the method, are measured. The results are plotted graphically and unknown samples are read off against the curve. The actual amounts of sample, specific binder and antibody are optimized to give a dose response curve of adequate slope (and hence of adequate assay sensitivity) over the desired working range of the assay. The process of optimizing an assay is one familiar to those who practice radio-immunoassay and related procedures.

However, there is a restriction upon the quantities specific binder employed in the free analyte assay, in that the greater the quantity employed, the more the position of equilibrium between free analyte and analyte bound to natural binder is altered; that is, the more the concentration of free analyte, at equilibrium, is altered. To some extent, the use of a dose response curve will correct for this, in that the position of equilibrium is altered in a similar way in the unknowns and in the standard samples. However, as the serum standards and the patient samples will inevitably have differing amounts of natural binders (it is the differing amounts of natural binders in different patients sera that render a free analyte assay valuable), it is highly desirable that the fraction of analyte removed from the natural binder (in terms of the total analyte present on the natural binder) should be as small as possible. However, no general rules can be given in that the fraction of analyte which can permissibly be removed from the natural binder depends on the accuracy needed by the clinician in the particular test, the proportion of analyte normally present in the free state, the variability experienced in patients samples regarding concentrations of natural binders, and so on. It is, as it usually is in clinical assays, a matter of optimizing the assay for its intended purpose.

Arising out of this, it is preferred to use as small a quantity of high affinity specific binder as possible, consistent with obtaining a dose-response curve of adequate slope, so as to avoid perturbing the bound-free analyte equilibrium any more than is necessary.

The antibody

As noted above, the antibody must bind to the specific binder in such a way as to compete with the free analyte for binding sites thereon.

In order to prepare anti-idiotype antibodies, animals are injected with purified antibodies, either unmodified, or as an alum precipitate, or polymerized by linking with glutaraldehyde. An antiserum may be prepared in the normal manner from blood taken from the animal. Alternatively, spleen cells from immunized mice may be used to prepare hybridomas from which clones of monoclonal antibody producing cells can be isolated. The following literature references describe the operation of anti-idiotype antibodies.

Walker ID & Morahan G, Scand. J. Immunol. 1981, 13, 433—440.

Weaver M S, Sikora L & Levy JG.

Molecular Immunology 1982, 19, 105—117.

Potocynjak P et al. Science 1982, 215, 1637—1639.

In one embodiment of this invention, the antibody may be labelled. The labelling criteria are the same as those for the specific binder and are set out above under the heading.

Either the specific binder or the antibody is labelled, as previously noted. The other of these two reagents has properties which permits quick and easy measurement of the fraction of the labelled reagent which forms part of the specific binder/antibody complex. (For this purpose, the assumption is made that all specific binder not bound to the antibody becomes bound to the analyte.) In this connection there are two categories of assay:—

i) Homogeneous assays. In these, the fraction of the labelled reagent that forms part of the specific binder/antibody complex is not separated from the fraction of the labelled reagent that does not form part of that complex prior to measurement of the signal emitted by the label. This is practicable and may be advantageous for enzyme, chemiluminescent and fluorescent assay systems. But it is necessary that the signal emitted by that fraction of the label which forms part of the specific binder/antibody complex be in some way different from the signal emitted by that fraction of the label which does not form part of that complex. In this invention therefore, either the antibody may be chosen to modify the signal emitted by the fraction of a labelled specific binder which becomes bound to it, or the specific binder may be chosen to modify the signal emitted by the fraction of a labelled antibody (e.g. a labelled anti-idiotype antibody) which becomes bound to it.

ii) Heterogeneous assays. In these the fraction of the labelled reagent which forms part of the specific binder/antibody complex is separated from the fraction of the labelled reagent which does not form part of the complex prior to measurement of the signal emitted by one or other fraction. Radioassay systems are normally heterogeneous, and other assay systems may be also. In this case, either the specific binder or the antibody serves as a means for separation, and is generally used, either in the solid phase, or in a form which is readily transferred from the liquid to the solid phase.

Assay conditions

Suitable conditions for performing the method of this invention, including time and temperature of incubation, and pH of the assay medium, may be readily determined from a knowledge of conventional assays for the analyte. The reactants may be mixed in different ways:—

i) The specific binder and the antibody are added simultaneously or consecutively to the assay sample, whereby the free analyte and the antibody compete for reaction with the specific binder.

ii) The specific binder is added to the assay sample in a form bound to the antibody. Some of the specific binder is freed from the complex and becomes bound to the free analyte.

As noted above, the specific binder is often an antibody to the analyte. It is known that antibodies are divalent. It might be thought that a problem would arise because a molecule of antibody would react with both a molecule of the analyte and a molecule of the antibody. If this were to happen to any great extent, this could reduce the sensitivity of the assay. However, this problem does not appear to arise to any

5

significant degree in practice. The problem can be avoided, if necessary, by rendering the antibody monovalent, e.g. by splitting the antibody into two smaller monovalent molecules (e.g. Fab fragments).

The method of this invention is envisaged mainly as an equilibrium approach to assay. However, some reactions may be rather slow to reach equilibrium, particularly where the analyte and/or the antibody is a large molecule. Also, the assay involves competition between two different reactions (the analyte/specific binder binding reaction and the specific binder/antibody binding reaction) which will have different reaction rates. If necessary, the method of the invention can be adapted to a kinetic or non-equilibrium approach.

The method of the invention can be adapted to both homogeneous and heterogeneous assays. In heterogeneous assays, separation of the fraction of specific binder bound to the antibody from the fraction not so bound usually involves separation of a solid from a liquid phase, and this may be effected by conventional means, e.g. centrifuging. Measurement of the signal emitted by the bound and/or the unbound fraction may also be effected by conventional means. In order to convert the resulting measurement into a value for the concentration of free analyte in the assay sample, recourse may be had to a set of standards containing different known concentrations of free analyte. The measurements obtained for the standards may be plotted on a graph of signal against free analyte concentration, and the free analyte concentration of the assay sample read off the graph.

Example

A standard curve for free 7-aza-8-oxo-9-(4-hydroxy-5-iodo-3-nitrophenyl) nonanoic acid (NIP-cap) in albumin solution

The following reagents were employed:

1. Buffer; which was 10 mmol/l phosphate, pH 7.4, containing 1 g/l sodium azide.

2. The analyte; which was 7-aza-8-oxo-9-(4-hydroxy-5-iodo-3-nitrophenyl) nonanoic acid hereafter designated NIP-cap. This was dissolved in buffer containing 4.5 g/l albumin. The analyte solutions were calibrated for free NIP-cap concentration by ultrafiltration dialysis as the reference method, a technique familiar to those skilled in the art.

3. The specific binder; which was a monoclonal antibody designated B1—8. The specific binder was produced by fusing myeloma cells with spleen cells from mice immunized with the hapten (4-hydroxy-5-iodo-3-nitrophenyl) acetyl. [M. Reth, G. J. Hammerling and K. Rajewsky, Eur. J. Immunol. 8, pp 393—400 (1978)]. The production of such monoclonal antibodies and their properties are matters well known to those familiar with the art.

The specific binder was coupled with solid-phase microscopic plastic particles. Such particles, the coupling process and the properties of binder coated plastic particles are matters familiar to those skilled in the art. The specific binder coupled particles were suspended in buffer to give a concentration of 0.2 mg of binder/l.

4. The antibody to the specific binder; which was a monoclonal antibody designated AC146 produced by fusing myeloma cells with spleen cells from mice immunized with B1—8. [M. Reth, T. Imamishi-Kari and K. Rajewsky, Eur. J. Immunol, 9, pp 1004—1014 (1979)]. The production of monoclonal anti-idiotype antibodies is a technique familiar to those skilled in the art, references demonstrating this art are: M. J. Nelles, L. A. Gill-Pazaris and A. Nisonoff, J. Exp. Med. 154, pp 1752—1763 (1981); J. F. Kerney, R. Barletta, Z. S. Quan and J. Quintans, Eur. J. Immunol. 11, pp 877—883 (1981); G. Morahan, J. Immunol. Methods 57 pp 165—170 (1983); P. Sanchez, C. Le Guern, L. Phalente, E. Barbier, G. Buttin and P. A. Cazenave, Molecular Immunology 19 pp 885—892 (1982). The antibody AC146 binds to an idiotope on the specific binder such that its binding to the specific binder can be inhibited by derivatives of (4-hydroxy-5-iodo-3-nitro-phenyl) acetyl such as NIP-cap [M. Reth, T. Imanishi-Kari and K. Rajewsky, Eur. H, Immunol. 9 pp 1004—1013 (1979)].

25 µg of the antibody was labelled with $7,4.10^7$ Bg (2mCi) of $^{125}$I using Bolton Hunter reagent [A. E. Bolton and W. M. Hunter, Biochem. J. 133 pp 529—538 (1973)]. The labelled antibody was dissolved in buffer containing 1 g/l gelatin at a concentration which gave 25 Kcpm/ml when the radioactivity was measured. In the free analyte asssy 100 µl of the analyte solutions were mixed with 400 µl of labelled antibody and 500 µl of specific binder. The mixture was incubated for one hour at room temperature. The reaction mixtures were then centrifuged and the supernatant liquid decanted. The radioactivity remaining in the tubes (which is that portion of the radioactivity bound to the specific binder) was measured. The assay was calibrated using the free NIP-cap concentration in the immunoassay system. This was calculated from the free NIP-cap concentration of the analyte solution (measured by ultrafiltration) by adjusting for the effect of dilution with reagent using the value of the association constant of NIP-cap and albumin.

The following results are typical of those obtained using this method.

| Free NIP-cap concentration (mol/l) | Proportion of radioactivity bound to specific binder (%) |
|---|---|
| Zero | 26.5 |
| $25.6 \times 10^{-9}$ | 21.2 |
| $51.6 \times 10^{-9}$ | 17.2 |
| $104 \times 10^{-9}$ | 13.6 |
| $208 \times 10^{-9}$ | 9.8 |

Example 2

Assay for free NIP-cap in solutions containing different concentrations of albumin

This experiment was designed to compare the values obtained using the method of this invention to the measurement of free NIP-cap concentrations with those obtained using the (ultrafiltration dialysis) reference method.

NIP-cap was dissolved in buffer containing human serum albumin and diluted with labelled antibody and solid phase antibody. The free NIP-cap concentrations of those solutions were measured by the method of Example 1. The values so obtained were compared to those calculated from ultrafiltration of the analyte solution; this is a method which is considered by those familiar with these matters to be a suitable method for the accurate measurement of the concentration of free analytes.

The following results are typical of those obtained in such comparisons:

| Albumin concentration (g/l) | Total NIP-cap concentration (mol/l) | Proportion of radioactivity bound to specific binder (%) | Free NIP-cap concentration by method of invention (mol/l) | Free NIP-cap concentration by ultrafiltration dialysis (mol/l) |
|---|---|---|---|---|
| 0.9 | $4.8 \times 10^{-7}$ | 11.3 | $155 \times 10^{-9}$ | $125 \times 10^{-9}$ |
| 0.9 | $2.4 \times 10^{-7}$ | 16.6 | $60 \times 10^{-9}$ | $57 \times 10^{-9}$ |
| 0.9 | $1.2 \times 10^{-7}$ | 20.1 | $30 \times 10^{-9}$ | $30 \times 10^{-9}$ |
| 0.9 | $0.6 \times 10^{-7}$ | 22.7 | $15 \times 10^{-9}$ | $16 \times 10^{-9}$ |
| 0.225 | $2.4 \times 10^{-7}$ | 11.9 | $140 \times 10^{-9}$ | $142 \times 10^{-9}$ |
| 0.225 | $1.2 \times 10^{-7}$ | 16.1 | $65 \times 10^{-9}$ | $71 \times 10^{-9}$ |
| 0.225 | $0.6 \times 10^{-7}$ | 18.6 | $40 \times 10^{-9}$ | $39 \times 10^{-9}$ |

The correlation coefficient of the free NIP-cap concentrations as measured by the two methods was 0.98. It is evident from these results that there is close agreement between the method of this invention and ultrafiltration dialysis for the measurement of free NIP-cap concentrations, even when the analyte binding protein varies in concentration by as much as four-fold. This demonstrates the suitability of the method of this invention for the measurement of free analytes.

**Claims**

1. A competitive method of determining the concentration of the free portion of an analyte which is a member of a specific binding pair consisting of the analyte and a specific binder therefor, said free portion of the analyte being present in a biological fluid which also contains a portion of the analyte bound to one or more natural binders for the analyte, the bound and free portions of the analyte being in equilibrium with one another, by:—

a) forming a mixture of a sample of the biological fluid with an amount of the specific binder for the analyte and with a monoclonal antibody to the specific binder which competes with the analyte for binding sites on the specific binder, the amount of said specific binder being insufficient to substantially affect said equilibrium, at least one of said specific binder and said antibody being labelled:—

b) maintaining said mixture for a time to permit the free portion of the analyte and the antibody to compete for binding sites on the specific binder and become bound thereto in proportions which depend on the concentration of the free portion of the analyte present in the sample;

c) measuring the amount of the specific binder bound to the antibody, and/or the amount of the specific binder not bound to the antibody; and

d) using the measurement to determine the concentration of free analyte in the biological fluid.

2. A method as claimed in claim 1, wherein the antibody is an anti-idiotype antibody.

3. A method as claimed in claim 1 or claim 2, wherein the antibody is labelled.

4. A method as claimed in claim 3, wherein the specific binder is used in an insoluble form.

5. A method as claimed in any one of claims 1 to 3, wherein the specific binder is an antibody to the analyte.

6. A method as claimed in claim 5, wherein the specific binder is a monoclonal antibody.

7. An assay kit for use in the method of any one of claims 1 to 6, for determining the concentration of the free portion of an analyte present in a biological fluid which also contains a portion of the analyte bound to one or more natural binders for the analyte, the bound and free portions of the analyte being in equilibrium with one another, comprising a supply of a specific binder for the analyte, and a supply of an antibody to the specific binder, at least one of the specific binder and the antibody being labelled.

8. A kit as claimed in claim 7, wherein the specific binder is in an insoluble form and the antibody is labelled.

9. A kit as claimed in claim 7 or claim 8 wherein the specific binder is a monoclonal antibody to the analyte and the antibody is a monoclonal anti-idiotype antibody to the specific binder.

**Patentansprüche**

1. Kompetitives Verfahren zur Bestimmung der Konzentration des freien Teiles eines zu analysierenden Stoffes, der ein Teil eines spezifisch bindenden Paares ist, das aus dem zu analysierenden Stoff und einem spezifischen Bindemittel dafür besteht, wobei der freie Teil des zu analysierenden Stoffes in einer biologischen Flüssigkeit vorhanden ist, die auch einen Teil des zu analysierenden Stoffes gebunden an einen oder mehrere natürliche Bindemittel für den zu analysierenden Stoff enthält, wobei die gebundenen und freien Teile des zu analysierenden Stoffes im Gleichgewicht miteinander sind, durch:

a) Bildung einer Mischung einer Probe der biologischen Flüssigkeit mit einer Menge des spezifischen Bindemittels für den zu analysierenden Stoff und mit einem monoklonalen Antikörper gegen das spezifische Bindemittel, der mit dem zu analysierenden Stoff um die Bindungsstellen an dem spezifischen Bindemittel konkurriert, wobei die Menge des spezifischen Bindemittels nicht ausreichend ist um wesentlich das Gleichgewicht zu beeinträchtigen, wobei mindestens eines von dem spezifischen Bindemittel und dem Antikörper markiert ist,

b) Beibehaltung der Mischung für eine Zeit, um die Konkurrenz des freien Teils des zu analysierenden Stoffes und des Antikörpers um die Bindungsstellen an dem spezifischen Bindemittel und die Bindung daran in Anteilen, die von der Konzentration des freien Teils des in der Probe vorhandenen zu analysierenden Stoffes abhängen, zu ermöglichen,

c) Messen der Menge des spezifischen Bindemittels, das an den Antikörper gebunden ist und/oder der Menge des spezifischen Bindemittels, das nicht an den Antikörper gebunden ist, und

d) Verwendung der Messung um die Konzentration des freien zu analysierenden Stoffes in der biologischen Flüssigkeit zu bestimmen.

2. Verfahren nach Anspruch 1, worin der Antikörper ein anti-idiotypischer Antikörper ist.

3. Verfahren nach Anspruch 1 oder 2, worin der Antikörper markiert ist.

4. Verfahren nach Anspruch 3, worin das spezifische Bindemittel in einer unlöslichen Form verwendet wird.

5. Verfahren nach einem der Ansprüche 1 bis 3, worin das spezifische Bindemittel ein Antikörper gegen den zu analysierenden Stoff ist.

6. Verfahren nach Anspruch 5, worin das spezifische Bindemittel ein monoklonaler Antikörper ist.

7. Untersuchungssatz zur Verwendung bei dem Verfahren nach einem der Ansprüche 1 bis 6 zur Bestimmung der Konzentration des freien Teiles eines in einer biologischen Flüssigkeit vorhandenen zu analysierenden Stoffes, die auch einen Teil des zu untersuchenden Stoffes gebunden an ein oder mehrere natürliche Bindemittel für den zu analysierenden Stoff enthält, wobei die gebundenen und freien Teile des zu analysierenden Stoffes im Gleichgewicht miteinander sind, das eine Zufuhr eines spezifischen Bindemittels für den zu analysierenden Stoff und eine Zufuhr eines Antikörpers gegen das spezifische Bindemittel umfaßt, wobei mindestens eines von dem spezifischen Bindemittel und dem Antikörper markiert ist.

8. Satz nach Anspruch 7, worin das spezifische Bindemittel in einer unlöslichen Form vorliegt und der Antikörper markiert ist.

9. Satz nach Anspruch 7 oder 8, worin das spezifische Bindemittel ein monoklonaler Antikörper gegen den zu analysierenden Stoff ist und der Antikörper ein monoklonaler anti-idiotypischer Antikörper gegen das spezifische Bindemittel ist.

## Revendications

1. Méthode compétitive pour déterminer la concentration de la portion libre d'un analyte qui est un élément d'une paire à liaison spécifique constituée de l'analyte et d'un liant spécifique de celui-ci, ladite portion libre de l'analyte étant présente dans un liquide biologique qui contient également une portion de l'analyte liée à un ou plusieurs liants naturels de l'analyte, les portions libre et liée de l'analyte étant en équilibre entre elles, en

a) formant un mélange d'un échantillon du liquide biologique avec une quantité du liant spécifique de l'analyte et avec un anticorps monoclonal dirigé contre le liant spécifique, qui entre en compétition avec l'analyte pour occuper les sites de liaison se trouvant sur le liant spécifique, la quantité dudit liant spécifique n'étant pas suffisante pour affecter sensiblement ledit équilibre, l'un au moins dudit liant spécifique et dudit anticorps étant marqué;

b) conservant ledit mélange pendant quelque temps pour permettre à la portion libre de l'analyte et à l'anticorps d'entrer en compétition pour occuper les sites de liaison se trouvant sur le liant spécifique et de s'y lier en des proportions qui dépendent de la concentration de la portion libre de l'analyte présente dans l'échantillon;

c) mesurant la quantité du liant spécifique lié à l'anticorps, et/ou la quantité du liant spécifique non lié à l'anticorps; et

d) utilisant la mesure pour déterminer la concentration d'analyte libre dans le liquide biologique.

2. Méthode telle que revendiquée dans la revendication 1, dans laquelle l'anticorps est un anticorps anti-idiotype.

3. Méthode telle que revendiquée dans la revendication 1 ou la revendication 2, dans laquelle l'anticorps est marqué.

4. Méthode telle que revendiquée dans la revendication 3, dans laquelle le liant spécifique est utilisé sous une forme insoluble.

5. Méthode telle que revendiquée dans l'une quelconque des revendications 1 à 3, dans laquelle le liant spécifique est un anticorps dirigé contre l'analyte.

6. Méthode telle que revendiquée dans la revendication 5, dans laquelle le liant spécifique est un anti-corps monoclonal.

7. Kit de dosage destiné à être utilisé selon la méthode de l'une quelconque des revendications 1 à 6, pour déterminer la concentration de la portion libre d'un analyte présent dans un liquide biologique qui contient également une portion de l'analyte liée à un ou plusieurs liants naturels de l'analyte, les portions libre et liée de l'analyte étant en équilibre entre elles, comprenant une provision d'un liant spécifique de l'analyte, et une provision d'un anticorps dirigé contre le liant spécifique, l'un au moins du liant spécifique et de l'anticorps étant marqué.

8. Kit tel que revendiqué dans la revendication 7, dans lequel le liant spécifique est sous une forme insoluble et l'anticorps est marqué.

9. Kit tel que revendiqué dans la revendication 7 ou la revendication 8, dans lequel le liant spécifique est un anticorps monoclonal dirigé contre l'analyte et l'anticorps est un anticorps anti-idiotype monoclonal dirigé contre le liant spécifique.

FIG.1

FIG.2

1

FIG.3